(19) Europäisches Patentamt
European Patent Office
Office européen des brevets

(11) EP 0 748 621 B1

(12) EUROPÄISCHE PATENTSCHRIFT

(45) Veröffentlichungstag und Bekanntmachung des Hinweises auf die Patenterteilung:
**03.09.2003 Patentblatt 2003/36**

(51) Int Cl.$^7$: **A61K 6/10**, C08G 77/04

(21) Anmeldenummer: **96108901.8**

(22) Anmeldetag: **04.06.1996**

# (54) Neue Katalysator/Vernetzer-Kompositionen, ein Verfahren zu deren Herstellung und deren Verwendung

New catalyst/crosslinker compositions and method for their preparation and use

Nouvelles compositions de catalyseurs/réticulants, leur procédé de préparation et utilisation

(84) Benannte Vertragsstaaten:
**BE DE ES GB IT NL**

(30) Priorität: **16.06.1995 DE 19521803**
**25.07.1995 DE 19527101**

(43) Veröffentlichungstag der Anmeldung:
**18.12.1996 Patentblatt 1996/51**

(73) Patentinhaber: **GE Bayer Silicones GmbH & Co. KG**
**40699 Erkrath (DE)**

(72) Erfinder:
- **Friebe, Robert, Dr.**
  **51373 Leverkusen (DE)**
- **Schwabe, Peter, Dr.**
  **51375 Leverkusen (DE)**
- **Voigt, Reiner**
  **51377 Leverkusen (DE)**

(56) Entgegenhaltungen:
**WO-A-86/03120** **GB-A- 2 163 765**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue Katalysator/Vernetzer-Kompositionen, ein Verfahren zu deren Herstellung und deren Verwendung in kondensationsvernetzenden Silikon-Pasten, insbesondere im Dentalbereich.

Silikon-Pasten sind in der Zahnheilkunde als Abformmassen weit verbreitet und werden üblicherweise in Form von 2-Komponenten-Systemen eingesetzt. Im allgemeinen besteht das System aus einem mit Füllstoffen und gegebenenfalls weiteren Hilfs- und Zusatzstoffen vermischten Silikonpolymer auf Basis eines endständige Hydroxylgruppen aufweisenden Polydimethylsiloxans, welches je nach Zusammensetzung in verschiedenen Konsistenzen erhältlich ist, und einer Härterkomponente, bei der es sich üblicherweise um ein Umsetzungsprodukt von Metallcarboxylaten, insbesondere Dialkylzinndicarboxylaten, und Kieselsäureestern als Katalysator/Vernetzer-Komposition handelt.

Diese beiden Komponenten werden vor der Anwendung miteinander homogen gemischt und vernetzen bei Raumtemperatur innerhalb von 2 - 5 Minuten infolge einer Polykondensationsreaktion zu einem Silikonelastomeren und geringen Mengen Alkohol, die langsam aus dem Material herausdiffundieren.

Die Verwendung organischer Zinnverbindungen bzw. von den Umsetzungsprodukten organischer Zinnverbindungen mit Kieselsäureestem als Katalysatoren in kondensationsvernetzenden Polysiloxanmassen wurde in der Literatur, z.B. W. Noll, Chemie und Technologie der Silikone, Verlag Chemie, Weinheim, 2. Auflage 1964, S. 339- 340 eingehend beschrieben. In DAS 1 167 527, DD-B 83 248 und DOS 2 524 000 werden z.B. Umsetzungsprodukte von Dialkyzinndicarboxylaten mit Alkoxysilanen beschrieben. Aus DAS 1 104 705 sind zudem Umsetzungsprodukte von Dialkyzinnoxiden mit Organosiloxanen und gegebenenfalls Organoalkoxysilanen, wie beispielsweise Methyltriethoxysilan, bekannt. Diese Umsetzungsprodukte können als weitere Zusätze organische Säuren enthalten. Die an sich gute Reaktivität dieser Umsetzungsprodukte wird bekanntermaßen durch den Zusatz solcher Verbindungen verbessert.

Geeignete Katalysator/Vernetzer-Kompositionen können Zusätze von speziellen Isoparaffinen, wie 2,2,4,4,6,6,8-Heptamethylnonan und 2,2,4,4,6,6,8,8,10-Nonamethylundecan, enthalten (siehe US 4 891 400 und DOS 35 44 619). Diese Verbindungen wirken als Lösungsvermittler beim Einmischen der Härterkomponenten in die Silikonabformmassen, insbesondere in einer flüssigen Darreichungsform, womit schnell eine gute Homogenität der angemischten Paste erzielt wird. Zudem wird auch die Oberflächenspannung eines Flüssighärtertropfens in der Weise geändert, daß die tropfenweise Dosierung des Flüssighärters aus einer Tropfflasche auf einen Silikonpastenstrang erleichtert wird.

An die Eigenschaften dieser Katalysator/Vernetzer-Kompositionen werden in Abhängigkeit von dem betreffenden Einsatzgebiet besondere Anforderungen gestellt. Bei Verwendung dieser Produkte in der Zahnheilkunde müssen bestimmte anwendungstechnische Forderungen, insbesondere auch toxikologische Eigenschaften, erfüllt werden, wie z.B. eine große Reaktivität der Abformmasse mit Verarbeitungszeiten von 2 bis 5 Minuten und dabei möglichst kurzen Abbindezeiten von 5 bis 6 Minuten.

Bekanntermaßen zeichnen sich Methoxy- gegenüber Ethoxy-substituierten Silanen durch höhere Reaktivitäten aus. Außerdem weisen die vernetzten Abformmassen nach der Polykondensationsreaktion und 24 Stunden Lagerung eine geringere Schrumpfung durch das Abdiffundieren des kleineren Moleküls Methanol auf als nach dem Abdiffundieren von Ethanol. Damit erweist sich ein Anteil von Methoxysilanen in der Härterkomponente sowohl im Hinblick auf die Reaktivität als auch Dimensionsstabilität der Abformmassen, die ja eine wesentliche Voraussetzung für eine formgenaue Abbildung darstellt, als vorteilhaft. Auch die chemische Beschaffenheit der organischen Zinnverbindung hat Einfluß auf die Reaktivität der Härterkomponente. So sind Dibutylzinn-Verbindungen reaktiver als Dioctylzinn-Verbindungen.

Vergleicht man jedoch die toxikologischen Eigenschaften dieser Verbindungen, so zeigt sich, daß im Hinblick auf die geringere Toxizität gerade der Einsatz von Dioctylzinnverbindungen bevorzugt werden sollte (siehe P.J. Smith, Toxicological Data on Organotin Compounds, International Tin Research Institute, Fraser Road, Perivale, Greenford, Middlesex, I.T.R.I Publication No. 538, 1979):

$LD_{50}$ [mg/kg] (oral, Ratte)

| | |
|---|---|
| $(n\text{-}C_4H_9)_2SnO$ | 487-520 |
| $(n\text{-}C_8H_{17})_2SnO$ | >4000 |
| $(n\text{-}C_4H_9)_2SnCl_2$ | 112-219 |
| $(n\text{-}C_8H_{17})_2SnCl_2$ | >4000 |
| $(n\text{-}C_4H_9)_2Sn(OOCC_{11}H_{23})_2$ | 175 |
| $(n\text{-}C_8H_{17})_2Sn(OOCC_{11}H_{23})_2$ | >6000 |

Eine weitere wichtige anwendungstechnische Forderung an die Katalysator/Vemetzer-Komposition ist ihre Reaktionsstabilität und dabei insbesondere ihr Langzeitverhalten. Die Kieselsäureester und die organischen Zinnverbindungen werden üblicherweise unter Inertgasatmosphäre bei erhöhten Temperaturen umgesetzt und je nach Reaktivität des Produktes der eine oder andere Bestandteil nachgesetzt, um die geforderte Anfangsreaktivität der Härterkomponente zu erhalten. Vielfach stellt sich innerhalb von drei bis sechs Monaten nach der Herstellung ein chemisches Gleichgewicht ein und die Härterkomponente hat erst dann fiir die Anwendung die richtige Reaktivität.

Vor der Anwendung z.B. beim Zahnarzt müssen die Silikon-Abformmassen mit dem Härter innerhalb von 30 Sekunden homogen vermischt werden. Deshalb darf die einzusetzende Flüssighärtermenge nicht zu groß sein und muß sich gut untermischen lassen. Zum anderen darf sich nicht durch die Zumischung des Härters die Konsistenz der Abformmassen wesentlich ändern.

Die nach dem Stand der Technik bekannten Katalysator/Vernetzer-Kompositionen erfüllen nicht alle der gestellten Anforderungen. Es bestand daher die Aufgabe, geeignete Katalysator/Vemetzer-Kompositionen, insbesondere für eine Anwendung in der Zahnheilkunde, bereitzustellen, die die bekannten Nachteile bestehender Kompositionen nicht mehr aufweisen. Daher sollen die Katalysator/Vernetzer-Kompositionen eine geringe Toxizität, eine gute Reaktivität, möglichst ohne Veränderung vom Zeitpunkt der Herstellung bis zum Verbrauch nach zwei bis drei Jahren und eine Dimensionsänderung der vemetzten Silikonabformmassen von unter 1,5%, gemessen nach DIN 24823 (ISO 4823, Pt. 7.7), aufweisen.

Überraschenderweise wurde gefunden, daß Katalysator/Vernetzer-Kompositionen, die Vinyltrialkoxysilane und Dialkylzinnoxide als essentielle Bestandteile enthalten nahezu alle Anforderungen erfüllen. Die erfindungsgemäßen Katalysator/Vernetzer-Kompositionen zeichnen sich durch eine geringe Toxizität, eine hohe Reaktivität bei guten Verarbeitungszeiten und schnellen Abbindezeiten und einer hohen Dimensionsstabilität der vulkanisierten Abformmassen aus.

Gegenstand der vorliegenden Erfindung sind somit Katalysator/Vernetzer-Kompositionen, enthaltend

a) mindestens ein Vinyltrialkoxysilan als Vernetzer,
b) mindestens ein Dialkylzinnoxid als Katalysator,
c) gegebenenfalls eine organische Säure oder ein Gemisch aus mehreren Säuren als Reaktionszeitregler,
d) gegebenenfalls inerte Lösungsmittel und
e) gegebenenfalls Farbstoffe und/oder Farbpigmente.

In einer bevorzugten Ausführungsform ist Komponente c) in der Katalysator/Vernetzer-Komposition enthalten.

Komponente a) im Sinne der Erfindung umfaßt alle gängigen Vinyltrialkoxysilane. Bevorzugt sind dabei Vinyltrialkoxysilane mit Alkoxy = $C_1$-$C_6$-Alkoxyresten.

Besonders bevorzugt ist das Vinyltrialkoxysilan a) Vinyltrimethoxysilan und/oder Vinyltriethoxysilan und/oder deren Teilhydrolysat. Geringe Zusätze von weiteren Kieselsäureestern, wie z.B. Polymethylsilicat, Tetramethylsilicat, Polyethylsilicat oder Tetraethylsilicat, können ebenfalls zugegen sein.

Komponente b) im Sinne der Erfindung umfaßt alle gängigen Dialkylzinnoxide. Dialkylzinnoxide im Sinne der Erfindung sind vorzugsweise Dialkylzinnoxide mit Alkyl = $C_1$-$C_{12}$-Alkylresten.

Besonders bevorzugt ist b) Dioctylzinnoxid ($(C_8H_{17})_2$ SnO). Ebenfalls bevorzugt als Komponente b) ist Dibutylzinnoxid oder Mischungen aus Dibutylzinnoxid und Dioctylzinnoxid.

Organische Säuren c) im Sinne der Erfindung sind vorzugsweise aliphatische und aromatische gesättigte und ungesättigte Mono- und Dicarbonsäuren sowie Sulfonsäuren. Die gute Reaktivität der erfindungsgemäßen Härterkomponenten kann durch den Zusatz solcher organischer Säuren verbessert werden. Beispiele für solche organischen Säuren c) sind 2-Ethylhexansäure, Benzoesäure, p-Toluolsulfonsäure und Dodecylsulfonsäure. Besonders geeignet ist Benzoesäure. Zusätze dieser Säure erzeugen zudem besonders aktivitätsstabile Härter.

Bei den erfindungsgemäßen Isoparaffinen d) handelt es sich vorzugsweise um Verbindungen der allgemeinen Formel

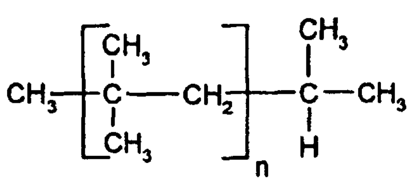

mit n = 2, 3, 4 oder 5.

Es ist selbstverständlich möglich Gemische von Isoparaffinen einzusetzen. Besonders bevorzugt als Komponente d) sind 2,2,4,4,6,6,8-Heptamethylnonan und/oder 2,2,4,4,6,6,8,8,10-Nonamethylundecan.

Farbstoffe und/oder Farbpigmente e) im Sinne der Erfindung sind alle nach dem Stand der Technik bekannten und verwendeten Farbstoffe oder Farbpigmente. In der erfindungsgemäßen Katalysator/Vernetzer-Komposition ist der Farbstoff e) vorzugsweise ein anorganisches oder organisches Farbpigment oder ein organischer Farbstoff. Besonders bevorzugt ist e) ein fettlöslicher Farbstoff.

Gegenstand der vorliegenden Erfindung sind zudem Katalysator/Vernetzer-Kompositionen die

a) mindestens ein Vinyltrialkoxysilan als Vernetzer,
b) mindestens ein Dialkylzinnoxid als Katalysator,
c) gegebenenfalls eine organische Säure oder ein Gemisch aus mehreren Säuren als Reaktionszeitregler,
d) gegebenenfalls inerte Lösungsmittel,
e) gegebenenfalls Farbstoffe und/oder Farbpigmente und zusätzlich noch
f) Füllstoffe und/oder Paraffine und/oder Vaseline und/oder Wachse und/oder Polydiorganosiloxane enthalten.

Die weiteren Zusatzstoffe f) erfindungsgemäßen Katalysator/Vernetzer-Kompositionen dienen hauptsächlich dazu, eine pastöse Darreichungsform herzustellen.

Die erfindungsgemäßen Katalysator/Vernetzer-Komposition enthält vorzugsweise

100 Gew.-Teile Vinyltrialkoxysilan a),
1 bis 100 Gew.-Teile Dialkylzinnoxid b),
0 bis 40 Gew.-Teile einer organischen Säure c),
0 bis 100 Gew.-Teile eines inerten Lösungsmittels d),
0 bis 10 Gew.-Teile Farbstoff e)
0 bis 300 Gew.-Teile Füllstoff und/oder Paraffine und/oder Vaseline und/oder Wachse und/oder Polydiorganosiloxane.

Von den einzelnen Komponenten können jedoch auch mehrere (verschiedene) Bestandteile vorhanden sein.

Gegenstand der vorliegenden Erfindung sind zudem Katalysator/Vernetzer-Kompositionen, erhältlich durch die Umsetzung von mindestens einem Vinyltrialkoxysilan a) und mindestens einem Dialkylzinnoxid b) und gegebenenfalls einer organischen Säure c).

Die Herstellung der erfindungsgemäßen Umsetzungsprodukte organischer Zinnverbindungen mit Alkoxysilanen findet üblicherweise bei erhöhten Temperaturen im Bereich von 50°C bis 180°C statt. In einem geeigneten Reaktionsgefäß werden dabei unter einer Inertgasatmosphäre die organische Zinnverbindung mit dem Alkoxysilan so lange erhitzt, bis eine klare Lösung entstanden ist. Es hat sich als vorteilhaft erwiesen, das Reaktionsgemisch gegebenenfalls noch einige Stunden bei erhöhten Temperaturen zu belassen. Die Umsetzung kann gegebenenfalls in Gegenwart einer organischen Säure erfolgen. Die Säure kann dabei sofort zu den Komponenten a) und b) gegeben und/oder nachdosiert werden.

Gegenstand der vorliegenden Erfindung ist zudem ein Verfahren zur Herstellung Komponente f)-freien Katalysator/Vernetzer-Kompositionen wonach mindestens ein Vinyltrialkoxysilan a) und mindestens ein Dialkylzinnoxid b) bei Temperaturcn von 0 bis 200°C, vorzugsweise 20 bis 150°C gegebenenfalls in Gegenwart weiterer Komponenten, ausgewählt aus c) bis e), umgesetzt werden.

Die Herstellung der erfindungsgemäßen Komponente f) enthaltenen Katalysator/Vernetzer-Komposition erfolgt nach gängigen Methoden. Dabei wird Komponente f) vorgelegt und a) und b) sowie gegebenenfalls c) bis e) unter Rühren nachdosiert.

Der Zusatz der weiteren erfindungsgemäßen Inhaltsstoffe c), d) und e) kann im Verlauf der Umsetzung bei erhöhten Temperaturen, als auch im Anschluß daran durch Einmischen bei Raumtemperatur erfolgen.

Gegenstand der vorliegenden Erfindung ist zudem die Verwendung der erfindungsgemäßen Katalysator/Vernetzer-Kompositionen in kondensationsvernetzenden Silikonmassen, z.B. im Bereich der Zahnheilkunde, Abform- und Vergußmassen, Dichtungs- und Beschichtungsmassen. Besonders bevorzugt ist der Einsatz in Zahnabformmassen und in Anwendungen, die für einen Kontakt mit Lebensmitteln bestimmt sind.

Gegenstand der vorliegenden Erfindung ist zudem die Verwendung der erfindungsgemäßen Katalysator/Vernetzer-Komposition in der Zahnheilkunde und in der Zahntechnik zur Kondensationsvernetzung von Massen auf Basis OH-endgestoppter Polydimethylsiloxane.

Die nachfolgenden Beispiele dienen der Erläuterung der Erfindung, ohne jedoch begrenzend zu wirken.

**Ausführungsbeispiele**

**Beispiele:**

A Allgemeine Beschreibung der Flüssighärter-Herstellung

In einem Dreikolben mit Rührer, Thermometer und Rückflußkühler wurden die in Tabelle 1 angegebenen Mengen an Alkoxysilan a) und an Dialkylzinnoxid b) und organischer Säure c) vorgelegt und unter Stickstoffatmosphäre ca. 4 Stunden bei einer Temperatur von ca. 120°C unter Rückfluß gerührt. Anschließend wurde dem Produkt, sofern dies schon nicht erfolgt ist, die organische Säure c) zugegeben und das Gemisch als Härter für die im folgenden beschriebene Abformmasse geprüft.

B Herstellung einer niedrigviskosen kondensationsvernetzenden Silikon-Abformmasse

In einem Mischkessel eines Butterfly-Rührers wurden 75 Gewichtsprozent eines OH-endgestoppten Polydimethylsiloxans mit einer Viskosität von 2000 mPa·s bei 23 °C, 24,7 Gewichtsprozent Calciumsilikat mit einer spezifischen Oberfläche von ca. 50 $m^2/g$ nach BET und 0,3 Gewichtsprozent eines organischen Farbpigmentes zu einer homogenen Abformmasse gemischt. Die Beurteilung fand ensprechend den unter C aufgeführten Bedingungen statt.

C Prüfung der Reaktivität und der Dimensionsänderung

Die nachfolgend beschriebenen Prüfungen wurden bei 23 ± 1 °C durchgeführt. Auf einem Anmischblock wurden 6,8 g der niedrigviskosen Abformmasse mit 0,26 ml des Flüssighärters mittels Spatel innerhalb von 30 Sekunden homogen gemischt. Ein Teil der angemischten Masse wurde in eine Aluminiumform von 20 mm Innendurchmesser und 6 mm Höhe gegeben und mit dem Spatel eine glatte Oberfläche gezogen. Diese Probe diente zur späteren Ermittlung der Abbindezeit.

Der auf dem Mischblock verbleibende Teil der angemischten Masse wurde im Abstand von 15 Sekunden mit dem Spatel angehoben und die Fließfähigkeit der Masse beobachtet. Wenn der Viskositätsanstieg durch die Vernetzungsreaktion soweit fortgeschritten war, daß die Masse nicht mehr vom Spatel ablief, ist das Ende der Verarbeitungszeit erreicht. Die Zeitspanne vom Mischbeginn bis Fließstopp wurde im Prüfprotokoll als Verarbeitungszeit festgehalten.

Die Abbindezeit wurde in der Weise ermittelt, daß von dem oben angegebenen Teil der Masse in der Aluminiumform eine Minute nach Beendigung der Verarbeitungszeit im Abstand von 15 Sekunden an einem Härteprüfgerät, beschrieben in DIN 53505, die Härte Shore A gemessen wurde bis zur Konstanz über drei Meßpunkte. 40 Minuten nach Mischbeginn wurde nochmals die Härte Shore A als Endwert bestimmt. Die Abbindezeit war die Zeitspanne von Mischbeginn bis zu dem Zeitpunkt, bei dem 80 % des Endwertes der Härte Shore A erreicht war.

Die Dimensions- oder lineare Maßänderung der Massen, bestehend aus 6,8 g Abformmasse und 0,26 ml Flüssighärter, wurde gemäß DIN 24 823 = ISO 4823 Pt. 7.7 durchgeführt.

Tabelle 1: Zusammensetzung und Prüfergebnisse der Flüssighärter.

| | Zusammensetzung des Flüssighärters | | | | | | Eigenschaften der Abformmasse mit Flüssighärter | | |
|---|---|---|---|---|---|---|---|---|---|
| | Alkoxysilan a) | Gew.-% | Zinnverbindung b) | Gew.-% | organ. Säure c) | Gew.-% | Verarbeitungszeit [min] | Abbindezeit [min] | Dimensionsänderung [%] |
| 1.[1)] | Tetraethoxysilan | 85,5 | Dibutylzinnoxid | 13,2 | 2-Ethylhexansäure | 1,3 | 7,5 | 21 | 1,26 |
| 2.[1)] | Tetraethoxysilan | 84,3 | Dibutylzinnoxid | 13,0 | 2-Ethylhexansäure | 2,7 | 13,5 | 27 | 1,43 |
| 3. | Vinyltriethoxysilan | 63,1 | Dioctylzinnoxid | 36,2 | Benzoesäure | 0,7 | 4 | 13 | 1,01 |
| 4. | Vinyltrimethoxysilan | 63,5 | Dioctylzinnoxid | 36,5 | | | 2 | 10 | 0,67 |
| 5. | Vinyltrimethoxysilan | 87,4 | Dioctylzinnoxid | 12,1 | Benzoesäure | 0,5 | 3 | 9 | 0,82 |
| 6.[2)] | Vinyltrimethoxysilan | 67,5 | Dioctylzinnoxid | 12,1 | Benzoesäure | 0,4 | 2,8 | 11 | 0,72 |

1) Vergleichsversuch

2) Flüssighärter enthält zusätzlich 20,0 Gew.-% 2,2,4,4,6,6,8,8,10 Nonamethylundecan

## Patentansprüche

**1.** Katalysator/Vernetzer-Kompositionen, enthaltend

a) mindestens ein Vinyltrialkoxysilan als Vernetzer,
b) mindestens ein Dialkylzinnoxid als Katalysator,
c) gegebenenfalls eine organische Säure oder ein Gemisch aus mehreren Säuren als Reaktionszeitregler,
d) gegebenenfalls inerte Lösungsmittel und
e) gegebenenfalls Farbstoffe und /oder Farbpigmente.

**2.** Katalysator/Vernetzer-Komposition nach Anspruch 1, **dadurch gekennzeichnet, daß** das Vinyltrialkoxysilan a) Vinyltrimethoxysilan und/oder Vinyltriethoxysilan und/oder deren Teilhydrolysat ist.

**3.** Katalysator/Vernetzer-Komposition nach einem der Ansprüche 1 bis 2, **dadurch gekennzeichnet, daß** das Dialkylzinnoxid b) Dioctylzinnoxid ist.

**4.** Katalysator/Vernetzer-Komposition nach einem der Ansprüche 1 bis 3, **dadurch gekennzeichnet, daß** die organische Säure c) eine aliphatische oder aromatische, gesättigte oder ungesättigte Mono-, Dicarbonsäure oder Sulfonsäure ist.

**5.** Katalysator/Vernetzer-Komposition nach einem der Ansprüche 1 bis 4, **dadurch gekennzeichnet, daß** die organische Säure c) Benzoesäure ist.

**6.** Katalysator/Vernetzer-Komposition gemäß den Ansprüchen 1 bis 5, **dadurch gekennzeichnet, daß** das inerte Lösungsmittel d) ein Isoparaffin der Formel

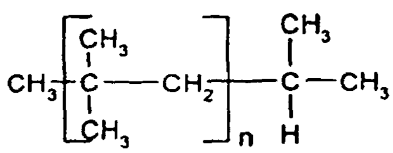

mit n = 2, 3, 4 oder 5 ist.

**7.** Katalysator/Vernetzer-Komposition gemäß den Ansprüchen 1 bis 6, **dadurch gekennzeichnet, daß** der Farbstoff e) ein anorganisches oder organisches Farbpigment oder ein organischer Farbstoff ist.

**8.** Katalysator/Vernetzer-Kompositionen nach einem der Ansprüche 1 bis 7, **dadurch gekennzeichnet, daß** diese zusätzlich f) Füllstoffe und/oder Paraffine und/oder Vaseline und/oder Wachse und/oder Polydiorganosiloxane enthalten.

**9.** Katalysator/Vernetzer-Kompositionen nach Anspruch 8, **dadurch gekennzeichnet, daß** diese

100 Gew.-Teile Vinyltrialkoxysilan a),
1 bis 100 Gew.-Teile Dialkylzinnoxid b),
0 bis 40 Gew.-Teile einer organischen Säure c),
0 bis 100 Gew.-Teile eines inerten Lösungsmittels d),
0 bis 10 Gew.-Teile Farbstoff e) und
0 bis 300 Gew.-Teile Füllstoff und/oder Paraffine und/oder Vaseline und/oder Wachs und/oder Polydiorganosiloxan f) enthalten.

**10.** Katalysator/Vernetzer-Komposition, erhältlich durch die Umsetzung von mindestens einem Vinyltrialkoxysilan a) und mindestens einem Dialkylzinnoxid b) und gegebenenfalls einer organischen Säure c).

**11.** Verfahren zur Herstellung einer Katalysator/Vernetzer-Komposition nach einem der Ansprüche 1 bis 7, **dadurch gekennzeichnet, daß** mindestens ein Vinyltrialkoxysilan a) und mindestens ein Dialkylzinnoxid b) bei Temperaturen von 0 bis 200°C, gegebenenfalls in Gegenwart von weiteren Komponenten, ausgewählt aus c) bis e), umgesetzt werden.

**12.** Verwendung der Katalysator/Vernetzer-Komposition nach einem der Ansprüche 1 bis 9 in kondensationsvernetzenden Polysiloxanmassen.

**13.** Verwendung der Katalysator/Vernetzer-Komposition nach einem der Ansprüche 1 bis 9 in der Zahnheilkunde und in der Zahntechnik zur Kondensationsvernetzung von Massen auf Basis OH-endgestoppter Polydimethylsiloxane.

## Claims

**1.** Catalyst/cross-linking agent compositions containing

a) at least one vinyltrialkoxysilane as cross-linking agent,
b) at least one dialkyltin oxide as catalyst,
c) optionally an organic acid or a mixture of plural acids as reaction time regulator,
d) optionally inert solvents and
e) optionally dyes and/or coloured pigments.

**2.** Catalyst/cross-linking agent composition according to Claim 1, **characterized in that** the vinyltrialkoxysilane a) is vinyltrimethoxysilane and/or vinyltriethoxysilane and/or a partial hydrolysate thereof.

**3.** Catalyst/cross-linking agent composition according to one of Claims 1 to 2, **characterized in that** the dialkyltin oxide b) is dioctyltin oxide.

**4.** Catalyst/cross-linking agent composition according to one of Claims 1 to 3, **characterized in that** the organic acid c) is an aliphatic or aromatic, saturated or unsaturated mono-, dicarboxylic acid or sulphonic acid.

**5.** Catalyst/cross-linking agent composition according to one of Claims 1 to 4, **characterized in that** the organic acid c) is benzoic acid.

**6.** Catalyst/cross-linking agent composition as per Claims 1 to 5, **characterized in that** the inert solvent d) is an isoparaffin of the formula

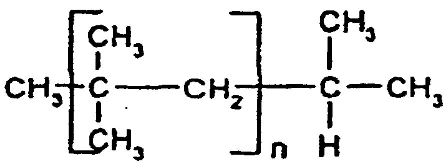

where n = 2, 3, 4 or 5.

**7.** Catalyst/cross-linking agent composition as per Claims 1 to 6, **characterized in that** the dye e) is an inorganic or organic coloured pigment or an organic dye.

**8.** Catalyst/cross-linking agent compositions according to one of Claims 1 to 7, **characterized in that** they additionally contain f) fillers and/or paraffins and/or petroleum jelly and/or waxes and/or polydiorganosiloxanes.

**9.** Catalyst/cross-linking agent compositions according to Claim 8, **characterized in that** they contain

100 parts by weight of vinyltrialkoxysilane a),
1 to 100 parts by weight of dialkyltin oxide b),
0 to 40 parts by weight of an organic acid c),
0 to 100 parts by weight of an inert solvent d),
0 to 10 parts by weight of dye e) and
0 to 300 parts by weight of filler and/or paraffins and/or petroleum jelly and/or wax and/or polydiorganosiloxane

f).

**10.** Catalyst/cross-linking agent composition obtainable via the reaction of at least one vinyltrialkoxysilane a) and at least one dialkyltin oxide b) and optionally an organic acid c).

**11.** Method for preparing a catalyst/cross-linking agent composition according to one of Claims 1 to 7, **characterized in that** at least one vinyltrialkoxysilane a) and at least one dialkyltin oxide b) are reacted at temperatures of 0 to 200°C optionally in the presence of further components selected from c) to e).

**12.** Use of the catalyst/cross-linking agent composition according to one of Claims 1 to 9 in polysiloxane materials which cross-link by condensation.

**13.** Use of the catalyst/cross-linking agent composition according to one of Claims 1 to 9 in dentistry and in dental technology for cross-linking materials based on OH-endstopped polydimethylsiloxanes by condensation.

## Revendications

**1.** Compositions de catalyseur / réticulant contenant

a) au moins un vinyltrialcoxysilane comme réticulant,
b) au moins un dialkyloxyde d'étain comme catalyseur,
c) éventuellement un acide organique ou un mélange de plusieurs acides comme régulateur de durée réactionnelle,
d) éventuellement un solvant inerte, et
e) éventuellement des colorants et/ou des pigments colorants.

**2.** Composition de catalyseur / réticulant selon la revendication 1, **caractérisée en ce que** le vinyltrialcoxysilane a) est un vinyltriméthoxysilane et/ou un vinyltriéthoxysilane et/ou leur hydrolysat partiel.

**3.** Composition de catalyseur / réticulant selon l'une des revendications 1 à 2, **caractérisée en ce que** le dialkyloxyde d'étain b) est le dioctyloxyde d'étain.

**4.** Composition de catalyseur / réticulant selon l'une des revendications 1 à 3, **caractérisée en ce que** l'acide organique c) est un acide mono-, dicarbonique saturé ou insaturé, aliphatique ou aromatique, ou l'acide sulfonique.

**5.** Composition de catalyseur / réticulant selon l'une des revendications 1 à 4, **caractérisée en ce que** l'acide organique c) est l'acide benzoïque.

**6.** Composition de catalyseur / réticulant selon l'une des revendications 1 à 5, **caractérisée en ce que** le solvant inerte d) est une isoparaffine de formule

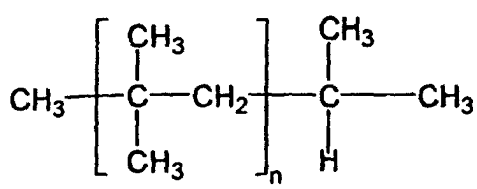

avec n = 2, 3, 4 ou 5.

**7.** Composition de catalyseur / réticulant selon l'une des revendications 1 à 6, **caractérisée en ce que** le colorant e) est un pigment colorant minéral ou organique ou un colorant organique.

**8.** Composition de catalyseur / réticulant selon l'une des revendications 1 à 7, **caractérisée en ce qu'**elle contient en outre f) des charges et/ou des paraffines et/ou de la vaseline et/ou des cires et/ou des polydiorganosiloxanes.

**9.** Composition de catalyseur / réticulant selon la revendication 8, **caractérisée en ce qu'**elle contient

100 parties en poids de vinyltrialcoxysilane a),
1 à 100 parties en poids de dialkyloxyde d'étain b),
0 à 40 parties en poids d'un acide organique c),
0 à 100 parties en poids d'un solvant inerte d),
0 à 10 parties en poids d'un colorant e) et
0 à 300 parties en poids d'une charge et/ou de vaseline et/ou de cire et/ou de polydiorganosiloxane f).

**10.** Composition de catalyseur / réticulant, pouvant être obtenue par réaction d'au moins un vinyltrialcoxysilane a) et d'au moins un dialkyloxyde d'étain b) et éventuellement d'un acide organique c).

**11.** Procédé de préparation d'une composition de catalyseur / réticulant selon l'une des revendications 1 à 7, **caractérisé en ce qu'**on fait réagir au moins un vinyltrialcoxysilane a) et au moins un dialkyloxyde d'étain b) à des températures de 0 à 200°C, éventuellement en présence d'autres composants, choisis parmi c) à e).

**12.** Utilisation de la composition catalyseur / réticulant selon l'une des revendications 1 à 9 dans des masses de polysiloxane réticulant par condensation.

**13.** Utilisation de la composition catalyseur / réticulant selon l'une des revendications 1 à 9 en médecine dentaire et en technique dentaire pour la réticulation par condensation de masses à base de polydiméthylsiloxane terminé par OH.